# EUROPEAN PATENT APPLICATION

(11) **EP 2 441 446 A1**
(43) Date of publication of application: **18.04.2012**
(21) Application number: 10013659.7
(22) Date of filing: 14.10.2010
(51) Int. Cl.: A61K 9/20, A61K 31/215

(54) **Using of superdisintegrants in cetyl myristate and/or cetyl palmitate formulations**

(71) Applicant: Deva Holding Anonim Sirketi, Kucukcekmece/Istanbul (TR)
(72) Inventor: Haas, Philipp Daniel, Kucukcekmece/Istanbul (TR); Firat, Omer Faruk, Kucukcekmece/Istanbul (TR); Kandemir, Levent, Kucukcekmece/Istanbul (TR); Ozbek, Mahmut, Kucukcekmece/Istanbul (TR); Koc, Fikret, Kucukcekmece/Istanbul (TR)

(57) **Abstract**

This invention is related to using of superdisintegrants in pharmaceutical or dietary supplement formulations of cetylated fatty acids, especially cetyl myristate or cetyl palmitate or combination of cetyl myristate and cetyl palmitate.

## Description

### Technical Field

This invention is related to using of superdisintegrants in pharmaceutical or dietary supplement formulations of cetyl myristate or cetyl palmitate or combination of cetyl myristate and cetyl palmitate. In this invention, cetyl myristate or cetyl palmitate or combination of cetyl myristate and cetyl palmitate are used as active pharmaceutical ingredients or dietary supplements.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstruc tive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses using of an superdisintegrant or mixtures of superdisintegrants in pharmaceutical or dietary supplement formulations of cetylated fatty acids, especially cetyl myristate or cetyl palmitate or combination of cetyl palmitate and cetyl myristate.

### Technical Problem

Cetyl myristate or cetyl palmitate or combination of cetyl myristate and cetyl palmitate can be used as active pharmaceutical ingredients (API) in pharmaceutical formulations in addition to excipient properties. However both cetyl myristate and cetyl palmitate are waxy ingredients and thus after ingestion they become gel thickness. It brings about difficulties for disintegration in gastrointestinal environment. Therefore it is needed that eligible formulation which has suitable disintegrating properties. Additionally, combination of cetyl myristate and cetyl palmitate is required high drug load and thus total pharmaceutical composition is weighted and volumed. Thus patients cannot easily ingest it. Ratherly low volume and low weight and having good disintegration properties are provided thanks to using of superdisintegrant instead of ordinary disintegrant.

### Solution to Problem

It is invented that gelling problem in gastrointestinal system is solved by use of superdisintegrants in formulations. Using of certain percentages provides to impede gelling in gastrointestinal system and provides having low volume and low weight pharmaceutical compositions.

### Description of embodiments

In accordance with this invention, superdisintegrants are used to prevent gelation of cetyl myristate or cetyl palmitate or combination of cetyl myristate and cetyl palmitate in gastrointestinal system.

Superdisintegrant is but not limited to croscarmellose sodium, sodium starch glycolate,crospovidone, L-hydroxypropyl cellulose, sodium carboxymethyl cellulose, mixtures thereof and the like.

The superdisintegrant or mixtures of superdisintegrants can be present in an amount in the range of 0.1 % to 10.0%, preferably 1% to 5%, more preferably 1.19% by weight of pharmaceutical composition. Most preferably disintegrant or mixtures of disintegrants are 5 mg of 420 mg in toto formulation.

In accordance with present invention, the pharmaceutical composition of cetyl myristate and cetyl palmitate combination furtherly comprises binder,diluent/filler,plasticizer and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Diluents/fillers are, but not limited to, mannitol, sorbitol, xylitol, microcrystalline cellulose, silicified microcrystalline cellulose , hydroxypropyl methylcellulose, hydroxypropyl cellulose , pullulan and fast dissolving carbohydrates such as Pharmaburst™, mixtures thereof and the like.

Plasticizers are, but not limited to, polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil , acetylated monoglycerides, mixtures thereof and the like. Plasticizer is used to have hard granulate.

In another aspect,this invention provides a weight ratio of superdisintegrant or mixtures of superdisintegrants to total pharmaceutical composition from1:10 to 1:200. Preferably weight ratio is 1:84.

In another aspect, this invention provides a weight ratio of superdisintegrant or mixtures of superdisintegrants to active ingredient(s) from1:15 to 1:180. Preferably weight ratio is 1:70.

To obtain elegant formulation superdisintegrant or mixtures of superdisintegrants provide disintegration not more than four minutes. Test is performed with disc and media is water. If test is performed without disc superdisintegrant or mixtures of superdisintegrants provide disintegration not more than five minutes.

## Claims

1. A pharmaceutical or dietary supplement composition of cetyl myristate or cetyl palmitate or combination of cetyl myristate and cetyl palmitate **characterized in that** (a) superdisintegrant or mixtures of superdisintegrants are in the range of 0.1 % to 10.0%, preferably 1% to 5%, more preferably 1.19% by weight of pharmaceutical composition or (b) weight ratio of superdisintegrant or mixtures of superdisintegrants to total pharmaceutical composition from1:10 to 1:200, preferably weight ratio is 1:84.or (c) weight ratio of superdisintegrant or mixtures of superdisintegrants to active ingredient(s) is from1:15 to 1:180,preferably 1:70 and preferably further includes excipients .

2. As claimed in claim 1, most preferably superdisintegrant or mixtures of superdisintegrants are 5 mg in formulations of 420 mg in toto.

3. According to preceding claims, superdisintegrant is selected from the group consisting of croscarmellose sodium, sodium starch glycolate,crospovidone, L-hydroxypropyl cellulose, sodium carboxymethyl cellulose and mixtures thereof.

4. As claimed in claim 1, in pharmaceutical or dietary supplement compositions of cetyl myristate or cetyl palmitate or combination of cetyl myristate and cetyl palmitate superdisintegrant or mixtures of superdisintegrants provide a disintegration wherein if test is performed with disc and media is water disintegration is occured not more than four minutes and/or if test is performed without disc disintegration is occured not more than five minutes.

5. As claimed in claim 4, in pharmaceutical or dietary supplement compositions of cetyl myristate or cetyl palmitate or combination of cetyl myristate and cetyl palmitate superdisintegrant or mixtures of superdisintegrants provide a disintegration wherein if test is performed with disc and media is water disintegration is occurred in 2-3 minutes and/or if test is performed without disc disintegration is occured 3-4 minutes.
